# EUROPÄISCHE PATENTSCHRIFT

(11) **EP 0 760 641 B1**
(45) Veröffentlichungstag und Bekanntmachung des Hinweises auf die Patenterteilung: **04.11.1998**
(21) Anmeldenummer: 95915808.0
(22) Anmeldetag: 19.04.1995
(51) Int. Cl.: A61F 5/01

(54) **ORTHESE**
ORTHOSIS
ORTHESE

(30) Priorität: 26.05.1994 DE 4418382
(43) Veröffentlichungstag der Anmeldung: 12.03.1997
(73) Patentinhaber: Klopf, Michael, 97297 Waldbüttelbrunn (DE)
(72) Erfinder: Klopf, Michael, 97297 Waldbüttelbrunn (DE)
(74) Vertreter: Pöhner, Wilfried Anton, Dr.
(86) Internationale Anmeldenummer: DE9500545
(87) Internationale Veröffentlichungsnummer: WO9532691

(56) Entgegenhaltungen:
- WO-A-93/02644
- FR-A- 2 477 409
- US-A- 4 958 643

## Beschreibung

Die Erfindung bezieht sich auf eine Orthese zur Stützung des Fußes, insbesondere bei Lähmungen, die durch Schädigung des Peroneusmuskels oder des Peroneusnervs hervorgerufen werden, mit einer Wadenschiene und einer Fußauflage, die über dessen Drehachse im wesentlichen senkrecht zur Seitenfläche des Fußes steht und das eine Feder enthält. Eine solche Orthese ist aus der WO 93/02644 bekannt.

Es ist bekannt, bei bestimmten Lähmungen den Fuß durch eine Orthese zu fixieren, deren Wadenschiene und Fußauflage entweder starr und unbeweglich oder über Blattfedern miteinander verbunden sind. Derartige Lähmungen entstehen insbesondere bei Schädigungen des Peroneusmuskels bzw. des diesen steuernden Peroneusnervs, wie sie z. B. durch die Folgen eines Schlaganfalls bedingt sein können. Die Lähmung wirkt sich dahingehend aus, daß die betroffenen Personen den Fuß nicht mehr bewußt steuern können und dadurch der Fuß nach unten fällt und hierbei eine Drehung nach Innen vollführt, die als Inversion oder Supination bezeichnet wird. Aufgrund dieser Drehbewegung entsteht beim Auftreten die Gefahr einer Schädigung.

Orthesen zur Stützung des Fußes sind in mehreren Ausführungsformen bekannt. Bei einer Ausführungsform ist die Wadenschiene unbeweglich mit der Fußauflage verbunden, wobei die Fußauflage und die Wadenschiene einen Winkel von ca. 90° einschließen.

Hierdurch ist der Fuß starr fixiert. Der Nachteil hierbei ist, daß die Orthese nicht nur eine Drehung des Fußes nach innen verhindert, sondern auch eine Auf- und Abwärtsbewegung im Gelenk unmöglich macht, wodurch das Gehen sehr erschwert wird. Außerdem besteht die Gefahr der Versteifung des Knöchels, da dieser völlig starr fixiert ist. Bei einer anderen Ausführungsform ist die Fußauflage über ein seitiches Blattfedernpaket mit der Schiene verbunden. Somit ist eine Auf- und Abwärtsbewegung des Fußes zwar prinzipiell möglich, jedoch kann die Federkraft und der Federweg nicht auf die individuellen Bedürfnisse des Patienten angepaßt werden, da keine nennenswerten Verstell- und Einstellmöglichkeiten vorhanden sind. Zudem wird keine definierte Drehachse erzeugt.

Aus der Druckschrift WO 93/02644 ist eine Schiene mit einem Drehgelenk bekannt, das mit einer durch eine Spiralfeder erzeugten Kraft beaufschlagt ist. Sie wird für die Therapie bei Schäden im Fuß- und Knöchelbereich eingesetzt, ist jedoch als Gehilfe ungeeignet. Die Druckschrift US 4 958 643 beschreibt ein Scharniergelenk für orthopädische Schienen, das ebenfalls eine Feder enthält. Zur Unterstützung von Gehbewegungen ist auch diese Schiene nicht einsetzbar.

Hiervon ausgehend liegt der Erfindung das Problem zugrunde, eine Orthese zur Stützung des Fußes, insbesondere bei Beeinträchtigung des Peroneusmuskels oder des Peroneusnervs, so auszugestalten, daß der Fuß in der Weise fixiert wird, daß eine seitliche Drehbewegung verhindert wird, jedoch eine Auf- und Abbewegung des Fußes möglich ist und die Federkraft, die in Richtung der Aufwärtsbewegung der Fußspitze wirkt, an die individuellen Bedürfnisse des Patienten angepaßt werden kann.

Erfindungsgemäß wird das Problem dadurch gelöst, daß die Federkraft in Richtung der Aufwärtsbewegung der Fußspitze wirkt und sich in der Ruhestellung des Fußes mit seiner Gravitationskraft die Waage hält und die Fußauflage in einen Schuh einsetzbar ist. Die Orthese ist in folgender Weise angebracht: Die Wadenschiene liegt an der Wade an und ist über das Gelenk mit der Fußauflage, die vom Schuh mit umschlossen wird, verbunden.

Da die Fußmuskulatur des gelähmten Fußes nicht angespannt werden kann, fällt er nach unten und vollführt eine Drehung nach innen. Die Orthese verhindert zum einen eine seitliche Drehung des Knöchels, und zwar dadurch, daß das Gelenk nur in Auf- und Abwärtsbewegung des Fußes bewegt werden kann. Dies wird durch die Lage der Drehachse erreicht, die senkrecht auf der Seitenfläche des Fußes steht, so daß sie mit einer Drehachse des Knöchels zusammenfällt. Zum anderen wird das Herabfallen durch die dieser Bewegung entgegenwirkende Federkraft ausgeglichen, welche dabei so eingestellt ist, daß sie sich in der Ruhestellung des Fußes mit der Gravitationskraft des Fußes die Waage hält. Beim Gehen wird die Feder aus dieser Ruheposition ausgelenkt, was dazu führt, daß beim Anheben des Fußes sich dieser wieder in die Ruheposition zurückbewegt.

Da sich das Gelenk unmittelbar seitlich neben dem Knöchel befindet, kann der Knöchel bewegt werden, nur die seitliche Drehbewegung wird verhindert. Das Gehen des Patienten wird wesentlich erleichtert, weil der Knöchel nicht starr fixiert ist. Außerdem wird durch die Bewegung des Knöchels einer Gelenkversteifung entgegengewirkt.

Sinnvollerweise nimmt die Federkraft mit der Auslenkung der Feder überproportional zu. Dies hat den Vorteil, daß die Dynamik der Drehbewegung dem normalen Gehablauf ähnlicher ist und somit ein beschwerdefreieres Gehen ermöglicht wird.

In einer sinnvollen Ausgestaltung der Erfindung ist die Vorspannung der Feder veränderbar. Dadurch kann die Federkraft an die individuellen Erfordernisse des Patienten angepaßt werden. So brauchen z.B. Orthesen für Kinder oder Erwachsene nicht verschiedene Federn enthalten und können prinzipiell einheitlich gefertigt werden. Dessen ungeachtet besteht selbstverständlich trotzdem die Möglichkeit, verschieden starke Federn in das Gelenk einzusetzen.

Die Feder ist zweckmäßigerweise eine Spiralfeder, wobei der Mittelpunkt der Spirale auf der Drehachse liegt. Die Feder ist dann am inneren Ende an der Drehachse und am äußeren Ende an der Innenwand des Gelenks befestigt. Da es sich bei der Bewegung des Gelenks um eine Drehbewegung um eine Achse handelt, ist die konstruktive Lösung mit einer Spiralfeder am einfachsten.

In einer zweckmäßigen Ausführungsform läßt sich die Vorspannung der Spiralfeder dadurch verändern, daß das äußere Ende der Spiralfeder an verschiedenen Stellen befestigbar ist. Dadurch kann die Feder entweder mehr gespannt oder mehr entspannt werden. Der Vorteil dieser Lösung liegt darin, daß die Vorspannung der Feder sehr einfach und unkompliziert verändert werden kann.

Beispielsweise kann das äußere Ende der Spiralfeder in radialen Bohrungen befestigt werden, die in der Innenwand des Gelenkes angebracht sind, wodurch Stellen geschaffen werden, an denen das Ende der Feder sicher verankert werden kann.

In einer weiteren Ausgestaltung ist die Feder eine Schraubenfeder, deren Federkraft im wesentlichen tangential auf einen im wesentlichen radialen Stempel wirkt, der mit dem zur Fußauflage gehörenden Teil des Gelenkes gehört.
Damit wird ein Drehmoment erzielt, das in die selbe Richtung wirkt wie bei der Orthese mit Spiralfeder.

Die Vorspannung der Schraubenfeder kann im Gegensatz zu der Spiralfeder kontinuierlich verändert werden und zwar dadurch, daß eine Schraube den Endpunkt der Feder verschiebt und damit die Federlänge verändert.

Die Länge von Schraubenfeder und dazugehöriger Schraube kann dadurch verkürzt werden, daß die Schraube axial in der Feder verläuft. Als Anschlagsfläche der Feder kann beispielsweise der Schraubenkopf dienen, wobei das Hineindrehen der Schraube in das Gelenk durch einen ausreichend langen Schraubenzieher bewerkstelligt werden kann.

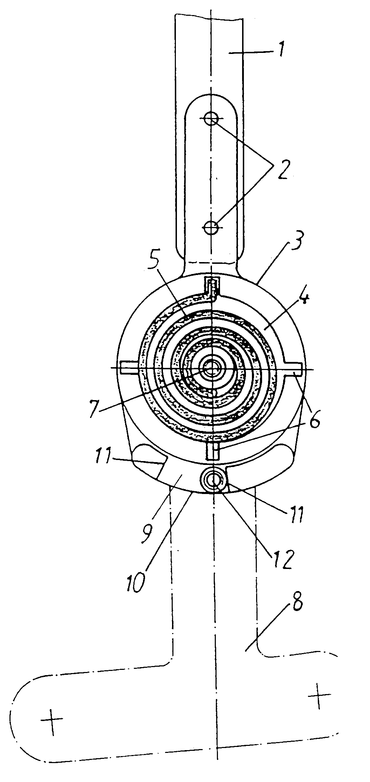

Ein Ausführungsbeispiel der Erfindung ist in der Zeichnung dargestellt und wird im folgenden näher beschrieben. Sie zeigt eine Seitenansicht der Orthese.

Die Zeichnung zeigt die Wadenschiene (1), die über zwei Nieten (2) mit dem Gelenk (3) verbunden ist. Das Gelenk (3) hat einen runden Querschnitt und hat einen Hohlraum (4) mit ebenfalls rundem Querschnitt. Die Spiralfeder (5) ist in dem Hohlraum (4) eingebettet und mit ihrem äußeren Ende in der oberen der vier radialen Bohrungen (6) befestigt, die in die Seitenwand des Gelenks (3) eingearbeitet sind. Die vier radialen Bohrungen (6) sind radial zum Mittelpunkt des Gelenks (3), weisen einen rechteckigen Querschnitt auf und schließen jeweils einen Winkel von 90° ein. Die Drehachse des Gelenks ist einerseits mit dem inneren Ende der Spiralfeder (5) als auch mit der Fußauflage (8) verbunden. Unterhalb des Gelenks (3) befindet sich eine Führungsschiene (9), die von oben durch die Außenwand des Gelenks, von unten durch ein zu dem Gelenk (3) konzentrischer Kreisbogen (10) und von links und rechts durch Drehanschlagsflächen (11) begrenzt ist. In der Führungsschiene (9) verläuft ein Zylinder (12) mit kreisförmigem Querschnitt, der mit der Fußauflage (8) verbunden ist. Dadurch ist die Drehbewegung der Fußauflage (8) auf den Kreisbogen (10) zwischen die beiden Drehanschlagsflächen (11) begrenzt.

## Patentansprüche

1. Orthese zur Stützung des Fußes, insbesondere bei Lähmungen, die durch Schädigung des Peroneusmuskels oder des Peroneusnervs hervorgerufen werden, mit einer Wadenschiene und einer Fußauflage, die über dessen Drehachse im wesentlichen senkrecht zur Seitenfläche des Fußes steht und das eine Feder enthält,
**dadurch gekennzeichnet,** daß
- die Federkraft in Richtung der Aufwärtsbewegung der Fußspitze wirkt und sich in der Ruhestellung des Fußes mit seiner Gravitationskraft die Waage hält
- und die Fußauflage (8) in einen Schuh einsetzbar ist.

2. Orthese nach Anspruch 1, **dadurch gekennzeichnet**, daß die Federkraft überproportional mit der Auslenkung der Feder (5) zunimmt.

3. Orthese nach einem der vorhergehenden Ansprüche, **dadurch gekennzeichnet**, daß die Vorspannung der Feder (5) veränderbar ist.

4. Orthese nach einem der vorhergehenden Ansprüche, **dadurch gekennzeichnet**, daß die Feder (5) eine Spiralfeder ist, wobei der Mittelpunkt der Spirale auf der Drehachse (7) liegt.

5. Orthese nach Anspruch 3 und 4, **dadurch gekennzeichnet**, daß die Vorspannung der Feder (5) dadurch veränderbar ist, daß ein Ende der Spiralfeder an verschiedenen Stellen befestigbar ist.

6. Orthese nach Anspruch 5, **dadurch gekennzeichnet**, daß die Stellen, an denen das äußere Ende der Spiralfeder (5) befestigbar ist, radiale Bohrungen (6) in der Innenwand des Gelenks (3) sind.

7. Orthese nach einem der Ansprüche 1 bis 3, **dadurch gekennzeichnet**, daß die Feder (5) eine Schraubenfeder ist, deren Federkraft im wesentlichen tangential zur Drehachse auf einen radialen Stempel wirkt.

8. Orthese nach Anspruch 3 und 7, **dadurch gekennzeichnet**, daß die Vorspannung der Feder (5) durch eine Schraube veränderbar ist, durch die die Federlänge in Richtung der Federkraft verkürzbar oder verlängerbar ist.

9. Orthese nach Anspruch 8, **dadurch gekennzeichnet**, daß die Schraube axial in der Feder (5) verläuft.

## Claims

1. Orthotic device to support the foot, in particular in the case of paralyses which are caused by damage to the peroneal muscle or the peroneal nerve, having a calf splint and a foot support, which via its rotation axis is essentially perpendicular to the lateral surface of the foot and which contains a spring,
**wherein,** that
- the spring power acts in the direction of the upward movement of the toes and counterbalances the foot in the resting position with its gravitational force,
- and said foot support (8) is insertable into a shoe.

2. Orthotic device according to claim 1, **wherein** the spring power increases superproportionally with the excursion of said spring (5).

3. Orthotic device according to one of the preceding claims, **wherein** the initial tension of said spring (5) is changeable.

4. Orthotic device according to one of the preceding claims, **wherein** said spring (5) is a spiral spring, whereby the centre of the spiral lies on the rotation axis (7).

5. Orthotic device according to claim 3 and 4, **wherein** the initial tension of spring (5) is changeable in that one end of the coil spring is affixable to different places.

6. Orthotic device according to claim 5, **wherein** the places to which the outer end of coil spring (5) is affixable are radial drill-holes (6) in the interior wall of hinge (3).

7. Orthotic device according to one of claims 1 to 3, **wherein** spring (5) is a helical spring the spring power of which acts essentially tangentially in relation to the rotation axis upon a radial bar.

8. Orthotic device according to claim 3 and 7, **wherein** the initial tension of spring (5) is changeable through a screw by means of which the spring length call be shortened or expended in the direction of the spring power.

9. Orthotic device according to claim 8, **wherein** the screw extends axially in the spring (5).

## Revendications

1. Orthèse destinée à soutenir le pied, notamment en cas de paralysie due à une lésion du muscle ou du nerf péronier, renfermant une attelle le long du mollet et un dispositif sur lequel repose le pied, placé au-dessus de l'axe de rotation de ce dernier et essentiellement perpendiculaire à sa face latérale, et un ressort,
**caractérisée en ce que**
- la force du ressort agit de sorte à faire remonter la pointe du pied et s'équilibre avec la force de la pesanteur du pied lorsque celui-ci est au repos
- et le dispositif sur lequel repose le pied (8) peut être placé dans une chaussure.

2. Orthèse selon la revendication 1**, caractérisée en ce que** la force du ressort augmente de manière surproportionnelle lorsque le ressort quitte sa position de repos (5).

3. Orthèse selon l'une quelconque des revendications précédentes, **caractérisée en ce qu'**il est possible de régler la précontrainte du ressort (5).

4. Orthèse selon l'une quelconque des revendications précédentes, **caractérisée en ce que** le ressort (5) est un ressort en spirale, le centre de la spirale coïncidant avec l'axe de rotation (7).

5. Orthèse selon les revendications 3 et 4 **caractérisée en ce que** la pré-tension du ressort (5) peut être modifiée par le fait qu'une extrémité du ressort en spirale peut être fixée à plusieurs endroits.

6. Orthèse selon la revendication 5 **caractérisée en ce que** les emplacements où l'extrémité extérieure du ressort en spirale (5) peut être fixée sont des alésages (6) radiaux prévus dans la paroi intérieure du système d'articulation (3).

7. Orthèse selon une des revendications 1 à 3, **caractérisée par le fait** que le ressort (5) est un ressort à boudin dont la force élastique est en grande partie tangentielle à l'axe de rotation et agit sur un piston radial.

8. Orthèse selon les revendications 3 et 7 **caractérisée par le fait** que la pré-tension du ressort (5) peut être modifiée par l'intermédiaire d'une vis qui permet de rallonger ou de raccourcir la longueur du ressort dans le sens de la force élastique.

9. Orthèse selon la revendication 8, **caractérisée par le fait** que la vis tourne de façon axiale dans le ressort (5).
